# EUROPEAN PATENT APPLICATION

(11) **EP 2 743 696 A1**
(43) Date of publication of application: **18.06.2014**
(21) Application number: 12822275.9
(22) Date of filing: 10.08.2012
(51) Int. Cl.: G01N 33/543, G01N 1/28, G01N 33/48, G01N 33/53

(54) **METHOD FOR PRETREATING BIOLOGICAL SAMPLE CONTAINING PROTEIN**

(30) Priority: 11.08.2011 JP 2011176272
(71) Applicant: Otsuka Pharmaceutical Co., Ltd., Tokyo 101-8535 (JP); National University Corporation Tokyo Medical and Dental University, Bunkyo-ku Tokyo 113-8510 (JP)
(72) Inventor: SASAKI, Sei, Tokyo 113-8510 (JP); OHMOTO, Yasukazu, Osaka-shi Osaka 541-0045 (JP); MORI, Toyoki, Osaka-shi Osaka 541-0045 (JP); IWATA, Fusako, Osaka-shi Osaka 541-0045 (JP); MURAGUCHI, Masahiro, Osaka-shi Osaka 541-0045 (JP)
(74) Representative: HOFFMANN EITLE
(86) International application number: PCT/JP2012/070570
(87) International publication number: WO 2013/022107

(57) **Abstract**

The present invention provides a method for pretreatment of a biological sample in the immunoassay of a protein contained in the biological sample. The method includes the steps of: (1) freezing the biological sample at a temperature higher than -80°C, in particular at -70°C or higher; (2) thawing the frozen biological sample; and (3) solubilizing the biological sample using a surfactant.

SEQUENCE LISTING

<110>  OTSUKA PHARMACEUTICAL CO., LTD.
<110>  NATIONAL UNIVERSITY CORPORATION TOKYO MEDICAL AND DENTAL UNIVERSITY

<120>  Method for pre-treating of biological sample cintaining a protein

<130>  P12-114

<150>  JP 2011-176272
<151>  2011-08-11

<160>  5

<170>  PatentIn version 3.4

<210>  1
<211>  271
<212>  PRT
<213>  Homo sapiens

<400>  1

<210>  2
<211>  30
<212>  DNA
<213>  Artificial

<220>
<223>  Forward primer \201ihAQP2N-F\201j

<400>  2
gggcatgcat gtgggagctc cgctccatag                                        30

<210>  3
<211>  22
<212>  DNA
<213>  Artificial

<220>
<223>  Reverse primer \201ihAQP2N-R\201j

<400>  3
cgtagttgta gaggagggag cc                                                22

<210>  4
<211>  22
<212>  DNA
<213>  Artificial

<220>
<223>  Forward primer \201ihAQP2C-F\201j

<400>  4
ccctgctctc tccataggct tc                                                22

<210>  5
<211>  29
<212>  DNA
<213>  Artificial

<220>
<223>  Reverse primer \201ihAQP2C-R\201j

<400>  5
ccaagctttc aggccttggt accccgtgg                                          29

## Description

### Technical Field

The present invention relates to a method for pretreatment of a biological sample in the immunoassay of a protein contained in the biological sample. The present invention further relates to an immunoassay for determining a protein contained in a biological sample pretreated by the above method.

### Background Art

In recent years, a method of measuring biomarkers has been used as a method for obtaining information useful for diagnosis of various diseases. A "biomarker" refers to "a characteristic that is objectively measured and evaluated as an indicator of normal biologic processes, pathogenic processes, or pharmacologic responses to a therapeutic intervention." (BIOMARKERS DEFINITIONS WORKING GROUP: BIOMARKERS AND SURROGATE ENDPOINTS: PREFERRED DEFINITIONS AND CONCEPTUAL FRAMEWORK, CLIN PHARMACOL THER 2001;69:89-95). More specifically, a biomarker is a material of biological origin contained in biological samples, for example, body fluids such as serum and urine, and tissues. A biomarker is used as an indicator for quantitatively detecting biological changes *in vivo.*

To measure biomarkers, immunoassays such as ELISA (enzyme immunoassay), RIA (radioimmunoassay), and immunochromatography are used. Such immunoassays can be easily performed by using assay kits.

On the other hand, the problem of false positives and false negatives may arise in the measurement of biomarkers. In particular, in the measurement of a membrane protein as a biomarker, because the site recognized by a detection antibody is covered with a membrane, the site may not sufficiently react with the antibody. Accordingly, convenient methods using assay kits as mentioned above cannot detect a biomarker with high accuracy, and a method that can solve this problem has been desired.

### Summary of Invention

### Technical Problem

An object of the present invention is to provide a method for pretreatment of a biological sample in the immunoassay of a protein contained in the biological sample, in particular a membrane protein having the above problem. More preferably, an object of the present invention is to provide a biological sample pretreatment method that can solve the problem of false positives and false negatives possibly arising in the immunoassay of a protein contained in the biological sample and that can increase measurement accuracy and/or measurement sensitivity.

Another object of the present invention is to provide an immunoassay for determining a protein contained in a biological sample pretreated by the above method, in particular a membrane protein.

### Solution to Problem

When a biological sample containing a protein such as an insoluble protein, in particular a membrane protein, is subjected to an immunoassay using the protein as a biomarker, the target protein is often solubilized using a surfactant before the immunoassay. The present inventors conducted extensive research to solve the above-mentioned problem in the immunoassay of such biological samples, and found that when a biological sample containing a protein, in particular an insoluble protein such as a membrane protein is once frozen at a temperature higher than -80°C, particularly at -70°C or higher, and then thawed, the above-mentioned problem that may occur in the immunoassay of a protein can be resolved, and measurement accuracy and/or measurement sensitivity can be increased.

The present invention has been accomplished by conducting further research based on this finding. The invention includes the following.

### (I) Methods for Pretreatment of a Biological Sample Containing a Protein

(I-1) A method for pretreatment of a biological sample in the immunoassay of a protein contained in the biological sample, the method comprising the steps of:
   (1) freezing the biological sample at a temperature higher than -80°C, particularly at -70°C or higher;
   (2) thawing the frozen biological sample; and
   (3) solubilizing the biological sample using a surfactant.
(I-2) A method for pretreatment of a biological sample in the immunoassay of a protein contained in the biological sample, the method comprising the steps of:
   (2') thawing the biological sample frozen at a temperature higher than -80°C, particularly at -70°C or higher; and
   (3) solubilizing the biological sample using a surfactant.
(I-3) The method for pretreatment as described in (I-1) or (I-2), wherein the biological sample is a body fluid of an animal including a human.
(1-4) The method for pretreatment as described in (I-3), wherein the body fluid is urine.
(I-5) The method for pretreatment as described in any one of (I-1) to (I-4), wherein the surfactant used in solubilization step (3) is a nonionic surfactant.
(I-6) The method for pretreatment as described in (I-5), wherein the nonionic surfactant is polyoxyethylene (10) octylphenyl ether.
(I-7) The method for pretreatment as described in any one of (I-1) to (I-6), wherein the immunoassay is ELISA.
(I-8) The method for pretreatment as described in any one of (I-1) to (I-7), wherein the protein is a membrane protein.
(I-9) The method for pretreatment as described in (I-8), wherein the membrane protein is aquaporin.

### (II) Immunoassay of a Protein Contained in a Biological Sample

(II-1) An immunoassay for determining a protein contained in a biological sample, wherein the biological sample is pretreated by the method as described in any one of (I-1) to (I-9).
(II-2) The immunoassay as described in (II-1), wherein the protein is a membrane protein.
(II-3) The immunoassay as described in (II-1) or (II-2), wherein the protein is aquaporin.
(II-4) The immunoassay as described in (II-1) to (II-3), which is an ELISA.

### Advantageous Effects of Invention

The present invention can provide a method for pretreatment of a biological sample in order to induce a sufficient immune response in the immunoassay of a protein, in particular a membrane protein, contained in the biological sample. Accordingly, the method of the present invention can measure a membrane protein contained in a biological sample with high accuracy and high sensitivity, which cannot be measured with sufficiently high accuracy and/or sensitivity when measured by using conventional immunoassays.

### Brief Description of Drawings

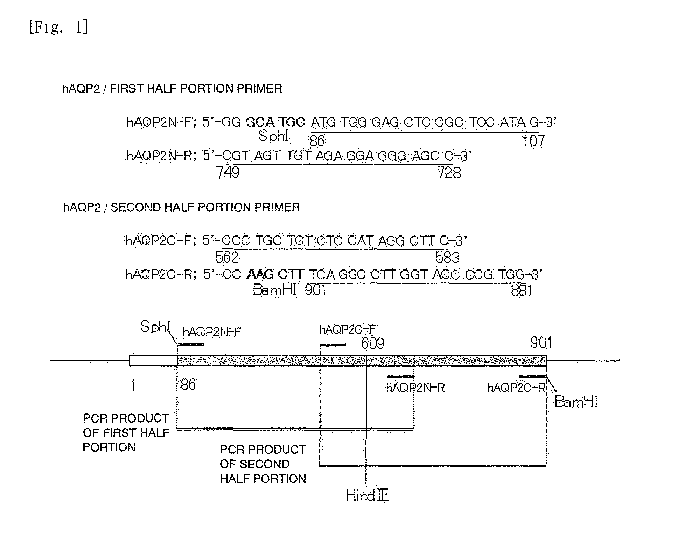

[FIG. 1] FIG. 1 shows the relationship between human AQP2 gene (hAQP2) and hAQP2/first half portion primers (hAQP2N-F, hAQP2N-R) or hAQP2/second half portion primers (hAQP2C-F, hAQP2C-R) used to subject the human AQP2 gene (hAQP2) to PCR (Reference Production Example 1 (2)).

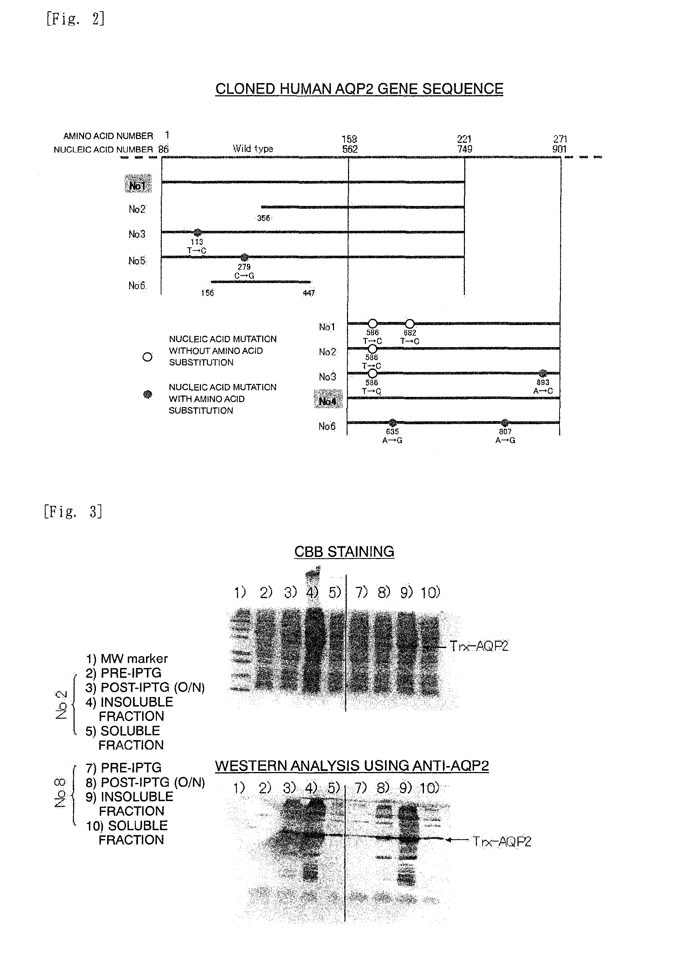

[FIG. 2] FIG. 2 shows a comparison in gene length and region between the sequences of hAQP2N (86-749) plasmid Nos. 1, 2, 3, 5, and 6 and hAQP2C (562-901) plasmid Nos. 1, 2, 3, 4, and 6 cloned in Reference Production Example 1 (2), and wild-type human AQP2 gene.
[FIG. 3] FIG. 3 shows the results of SDS-PAGE/CBB staining (upper figure) and Western blot analysis of two types of *Escherichia coli* (Nos. 2 and 8) using a human AQP2 synthetic peptide antibody (anti-human AQP2 rabbit serum, PherMingen) in Reference Production Example 1 (4), wherein pre-IPTG samples (before induction), post-IPTG samples (after induction), and insoluble fractions and soluble fractions that were obtained by solubilizing the induced *E. coli* were used.

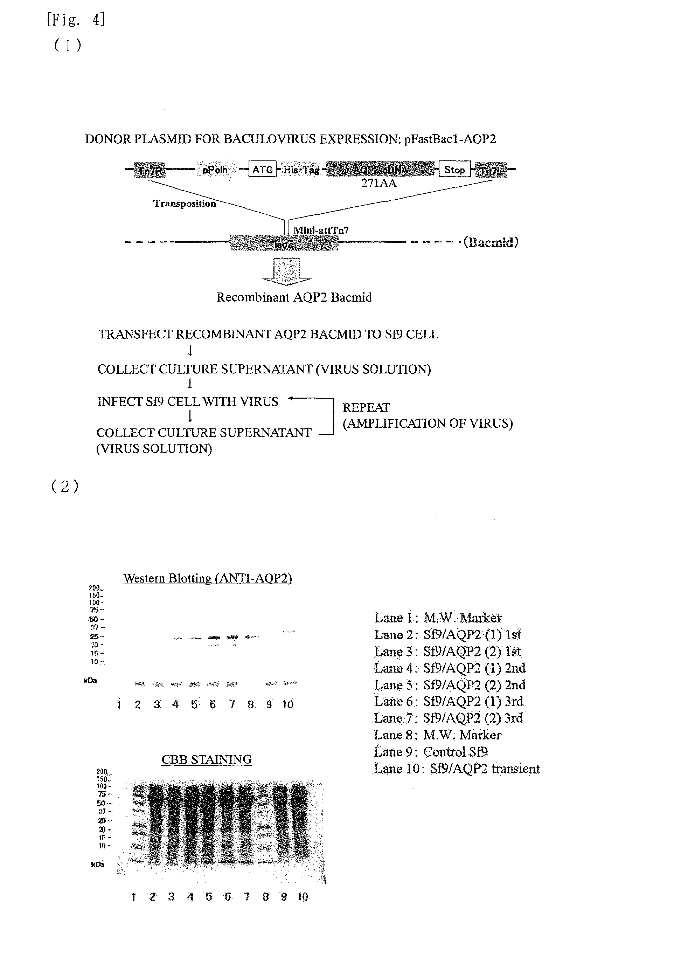

[FIG. 4] FIG. 4 (1) shows a schematic view of the production of His-tagged human AQP2 (hAQP2/His/Sf-9) expressed in Sf-9 insect cells in Reference Production Example 2, and FIG. 4 (2) shows the results of analysis thereof.

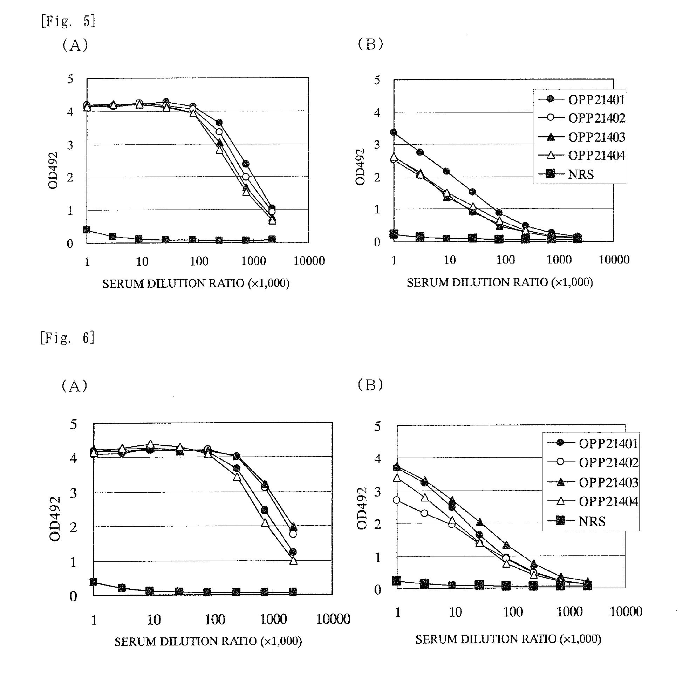

[FIG. 5] FIG. 5 (A) shows antibody titers of the serum (OPP21401-21404) after the third immunization and the control serum (NRS: normal rabbit serum) against the immunogen (hAQP2/Trx), and FIG. 5 (B) shows antibody titers of the same against Sf-9 cell-expressed hAQP2 (hAQP2/Trx/Sf-9) in Reference Production Example 3.
[FIG. 6] FIG. 6 (A) shows antibody titers of the serum (OPP21401-OPP 21404) after the seventh immunization and the control serum (NRS: normal rabbit serum) against the immunogen (hAQP2/Trx), and FIG. 6 (B) shows antibody titers of the same against Sf-9 cell-expressed hAQP2 (hAQP2/Trx/Sf-9) in Reference Production Example 3.

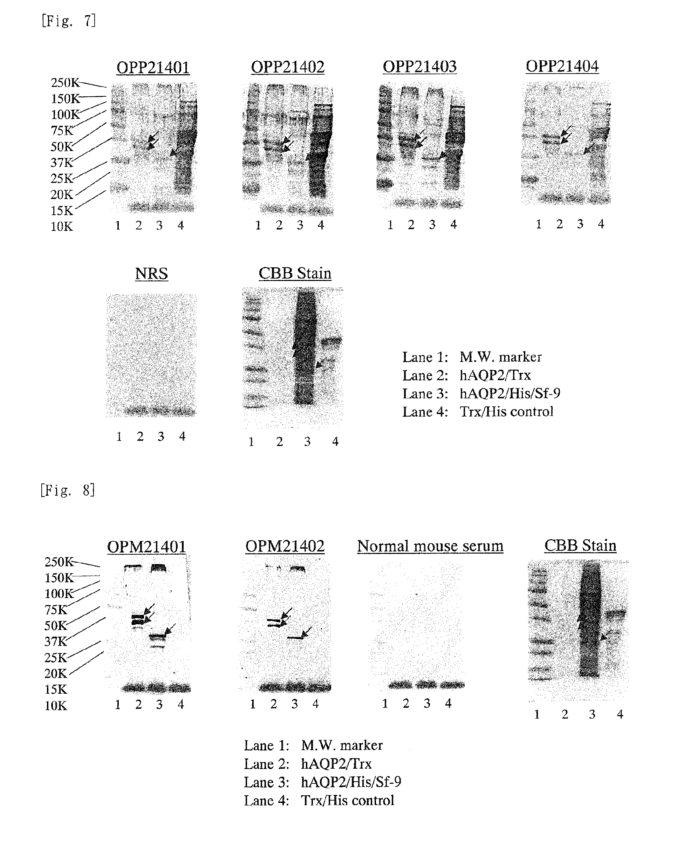

[FIG. 7] FIG. 7 shows the results of examination of the reactivity of the obtained polyclonal antibodies (OPP21401 - OPP21404) and the control serum (NRS: normal rabbit serum) with various antigens (hAQP2/Trx, hAQP2/Trx/Sf-9, and Trx/His control) by Western blotting in Reference Production Example 3.
[FIG. 8] FIG. 8 shows the results of examination of the reactivity of the obtained polyclonal antibodies (OPM21401, OPM 21402) and the control serum (NMS: normal mouse serum) with various antigens (hAQP2/Trx, hAQP2/Trx/Sf-9, and Trx/His control) by Western blotting in Reference Production Example 4.

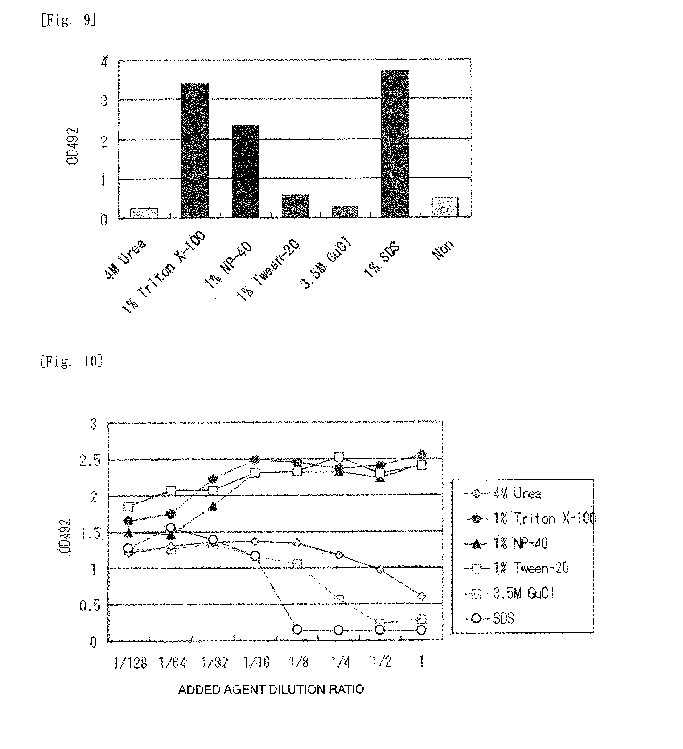

[FIG. 9] FIG. 9 shows the results of examination of the conditions that do not affect the assay system and under which AQP2 is solubilized from the membrane by using various solubilizers (Urea, Triton X-100, NP-40, Tween-20, guanidine hydrochloride (GuCl), and SDS) in Experimental Example (1) (1-1).
[FIG. 10] FIG. 10 shows the results of examination of stabilizers (Urea, Triton X-100, NP-40, Tween-20, guanidine hydrochloride (GuCl), and SDS) added to the first buffer (0.2% BSA/10 mM EDTA/0.2 M Tris-HCl (pH 8.0)) in Experimental Example 1 (1) (1-2).

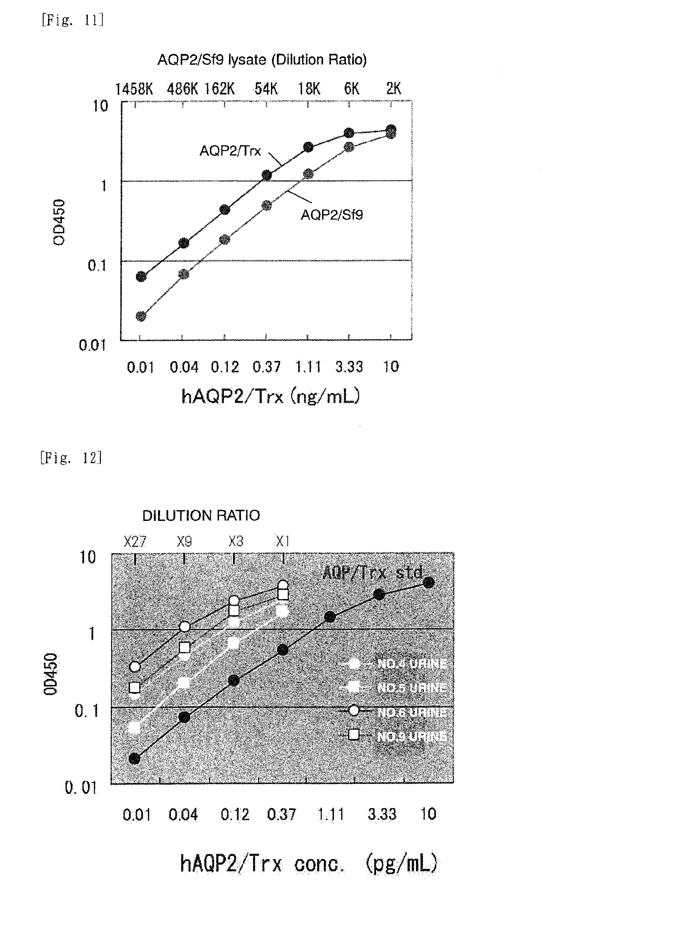

[FIG. 11] FIG. 11 shows standard curves obtained by performing ELISA using the monoclonal antibody (OPM21401) and using, as standard reference proteins, "Trx fusion AQP2 protein (AQP2/Trx)" produced by the method described in Reference Production Example 1 and "AQP2/Sf9" produced by the method described in Reference Production Example 2 (Experimental Example 1 (1) (1-4)). The dilution ratio shown on the upper abscissa indicates that a stock solution (AQP2/Sf9 lysate) prepared by suspending AQP2/Sf9 in PBS and solubilizing the suspended AQP2/Sf9 with an equal volume of solubilizer was diluted 2K-fold to 1458K-fold (i.e., 2 to 1458 x 1000 times).
[FIG. 12] FIG. 12 shows the results of ELISA using serially diluted 4 urine samples of healthy human volunteers (Nos. 4, 5, 6, and 9) as test samples, versus a standard curve of the standard protein (Trx fusion AQP2 protein) (Experimental Example 1 (1), (1-5)).

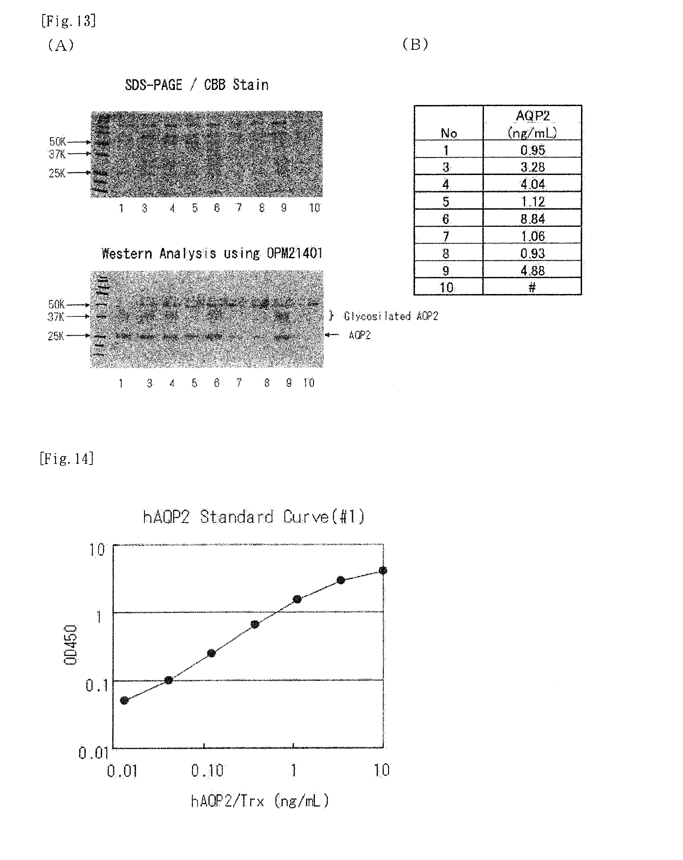

[FIG. 13] FIG. 13 (A) shows the results of measuring the amount of human AQP2 in urine samples of 10 healthy human volunteers using the monoclonal antibody (OPM21401) against human AQP2 (Reference Production Example 3) by Western blotting. FIG. 13 (B) shows the amount of human AQP2 in urine samples of the same 10 healthy human volunteers measured by the ELISA system (Experimental Example 1 (1) (1-6)).
[FIG. 14] FIG. 14 shows a standard calibration curve obtained by performing ELISA using, as a standard protein, "AQP2/Sf9" produced by the method described in Reference Production Example 2.

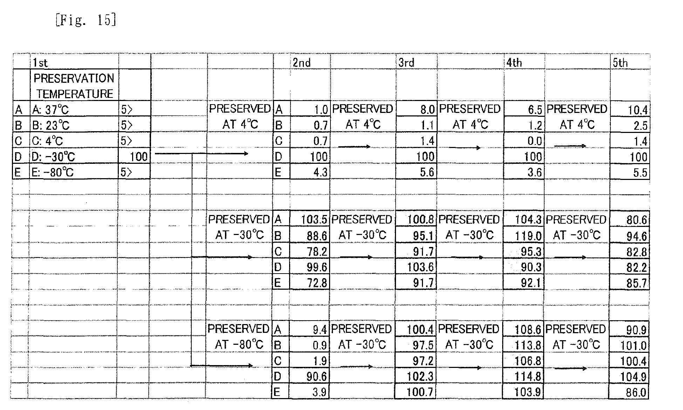

[FIG. 15] Fig. 15 shows the results of the first, second, third, fourth, and fifth tests in Experimental Example 2.

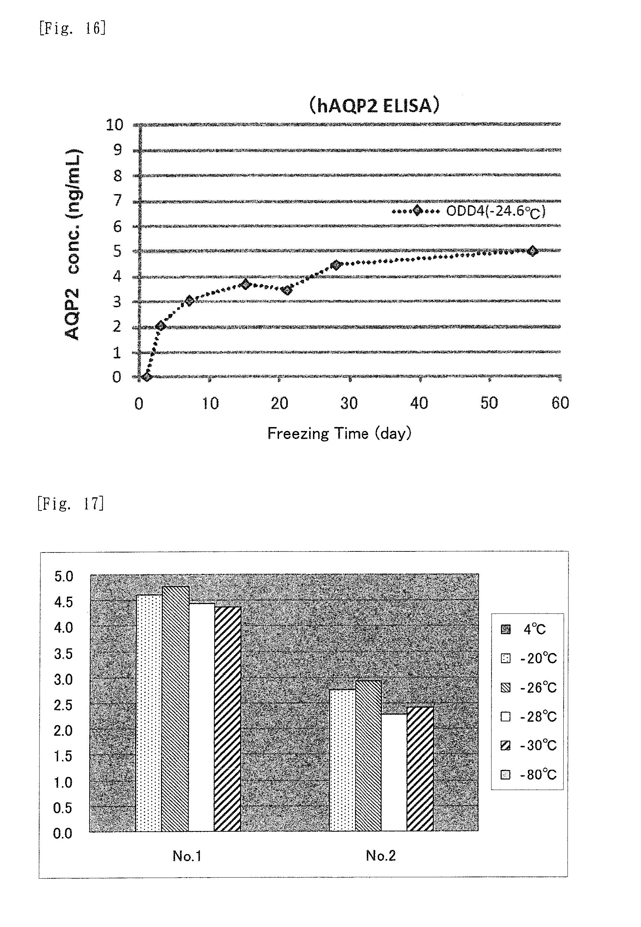

[FIG. 16] FIG. 16 shows the results of measuring the amount of AQP2 in human urine frozen at 20±5°C for 0 to 56 days, by using the human AQP2 ELISA established in Experimental Example 1, and investigating the relationship between the freezing period and the amount of AQP2 in the human urine (Experimental Example 3).
[FIG. 17] FIG. 17 shows the results of measuring the amount of AQP2 in urine samples (Nos. 1 and 2), which were obtained from two healthy persons and preserved in the dark for 2 weeks (14 days) at each of various temperatures
   (-80°C, -30°C, -28°C, -26°C, -20°C, 4°C), by using the human AQP2 ELISA established in Experimental Example 1, and investigating the relationship between the freezing temperature and the amount of AQP2 in human urine (Experimental Example 3). Description of Embodiments

### (I) Method for Pretreatment of a Biological Sample Containing a Protein

The method of the present invention is a pretreatment method for a biological sample in the immunoassay of a protein contained in the biological sample.

The immunoassay as used herein refers to a biochemical test method for determining the presence or amount of a target substance (a protein in the present invention) in a test sample by utilizing a reaction between an antigen and an antibody. Examples of immunoassays include, but are not limited to, enzyme immunoassay (EIA), radioimmunoassay (RIA), enzyme-linked immunosorbent assay (ELISA), agglutination, nephelometry, turbidimetry, and Western blot. ELISA is preferable.

The target biological sample of the present invention is not particularly limited in source and type, insofar as the sample contains a protein and can be subjected to immunoassay. For example, biological samples obtained from animals or plants can be used.

Although the type of source animal is not particularly limited, mammals including humans can be preferably used. Examples of mammals other than humans include rats, mice, guinea pigs, rabbits, monkeys, dogs, cows, horses, and the like. Humans are preferable as the source animal. Biological samples obtained from such animals include, for example, the following, each containing a protein, preferably a membrane protein: body fluids such as urine, blood, saliva, perspiration, mucus, lymph, amniotic fluid, and cerebrospinal fluid; and cells and tissues.

Although the type of source plant is not particularly limited, examples of plants include higher plants such as seed plants and ferns, and lower plants such as fungi, lichens and algae. Specific examples of biological samples obtained from such plants include various parts of plants (including processed products thereof, e.g., extracts), such as leaves, stems, roots, flowers, seeds, fruits, and fruit peels; secretions such as sap; and cells and tissues.

The biological sample is preferably a mammal-derived sample, particularly preferably a human biological sample. For the sake of convenience in collection, urine, saliva, and blood of mammals, in particular of humans, are more preferable. Urine is particularly preferable.

Such biological samples may be freshly obtained, or may be held for a certain period after collection, i.e., collected and stored for a certain period of time.

The method of collecting a biological sample is not particularly limited, insofar as the method does not affect the target protein, preferably membrane protein, contained in the biological sample.

Further, the collected biological sample can be subjected to the method of the present invention as is. Alternatively, a portion containing the target protein may be extracted or fractionated from the collected biological sample, and the extract may be subjected to the method of the present invention.

The target protein of the present invention is not particularly limited insofar as it is a protein contained in animals or plants as mentioned above. However, the target protein is preferably a membrane protein. The membrane protein is a protein that exists on a cell membrane or a vesicle membrane. Examples of membrane proteins include, but are not limited to, membrane transport proteins, Na⁺/K⁺-pumps, and ion channels. For example, the membrane protein is preferably aquaporin (Aquaporin:AQP), particularly preferably AQP2 that is one of the 13 types of human AQPs (see "Cell Membrane Water Channel, Aquaporin" The Medical Association of Nippon Medical School, 2009; 5 (2)).

AQP is one of the major membrane proteins that belong to the MIP (major intrinsic proteins) family (Agre P (2006), "The aquaporin water channels", Proc Am Thorac Soc 3 (1): 5-13; Agre P, et al., (1993), "Aquaporin CHIP: the archetypal molecular water channel" Am. J. Physiol. 265: (4 Pt 2) F463-76). Aquaporin exists in collecting duct luminal membranes and is a water channel that shuttles between the cell membrane surface and the intracellular vesicle membrane. Aquaporin selectively passes only water and thus relates to cellular water uptake (Gonen T, WalzT (2006), "The structure of aquaporins", Q. Rev. Biophys. 39 (4): 361-96). The amino acid sequence of aquaporin is already known. For example, the amino acid sequence of human AQP2 was registered in GenBank under accession No: AAB319999.1 (GI:685001)(SEQ ID NO: 1), and the nucleotide sequence encoding the amino acid sequence was registered in GenBank under accession No: AH 007818.3 (GI:14190630).

A feature of the method of the present invention is that the method comprises the following steps:
(1) freezing the biological sample at a temperature higher than -80°C, in particular at -70°C or higher (a freezing step);
(2) thawing the frozen biological sample (a thawing step); and
(3) solubilizing the biological sample using a surfactant (a solubilization step).

Each step is described below.

### (1) Freezing step

This is a step of freezing the biological sample at a temperature higher than -80°C.

The freezing method is not particularly limited insofar as the method does not adversely affect the protein contained in a biological sample, and the target biological sample can be frozen at a temperature higher than -80°C. The biological sample can be typically frozen by using a freezer that can perform freezing at the above-mentioned temperature (freezer temperature: higher than -80°C).

The freezing treatment temperature is not particularly limited insofar as it is higher than -80°C as described above. The temperature is preferably -70°C or higher, more preferably -50°C or higher, specifically -50°C to -10°C, even more preferably -40°C or higher, and specifically -40°C to -10°C. Still more preferably, the temperature is higher than -40°C, specifically higher than -40°C and lower than -10°C, more specifically in the range of -35°C to -15°C, and particularly preferably in the range of -30°C to -20°C.

The frozen biological sample may be subjected to the subsequent thawing step (2) immediately after step (1). Alternatively, the frozen biological sample once frozen may be maintained in a frozen state for a certain period of time. Although the period for which the sample is maintained in a frozen state is not particularly limited, it is preferably 1 hour to 1 week, and more preferably 12 hours to 24 hours.

The results of the Experimental Examples described below indicate that freezing of a biological sample for a certain period of time or longer can stabilize the immunoassay value of a protein obtained from the biological sample. Accordingly, to quantify the protein contained in a biological sample with high accuracy, it is preferable that the biological sample be maintained in a frozen state for a certain period of time or longer, preferably for about 2 weeks or longer. The freezing temperature conditions for maintaining the biological sample in a frozen state for about 2 weeks are preferably in the range of, for example, -30°C to -20°C.

### (2) Thawing step

This is a step of thawing the biological sample frozen in the above step.

The thawing method is not particularly limited insofar as the method does not adversely affect the protein contained in a biological sample and can thaw the frozen biological sample. Specific examples of such methods include, but are not limited to, a method of allowing a frozen biological sample in a container to stand at room temperature, a method of applying running water, a method of immersion in water, and a method of gradual thawing on ice.

The freezing and thawing treatments described above in (1) and (2) may be performed once, or repeated more than once. In view of treatment efficiency, each of the freezing and thawing treatments is usually performed once. However, as described in the Experimental Example described below, repetition of the freezing and thawing treatments does not significantly deteriorate immunoassay results, and appears not to adversely affect the protein to be measured. Therefore, repetitively performing freezing and thawing multiple times is not restricted. Accordingly, in a case where it is unknown whether the freezing treatment has been done, for example, when it cannot be confirmed whether the biological sample to be subjected to immunoassay has been frozen at a temperature higher than -80°C, in particular at -70°C or higher, freezing and thawing may be performed again.

Alternatively, when a biological sample previously subjected to step (1) is available (for example, when a sample subjected to step (1) by a third party is obtained), the present invention can be carried out by performing the following step (2') instead of steps (1) and (2):
(2') thawing a biological sample frozen at a temperature higher than -80°C, in particular at -70°C or higher.

Thus, in another aspect, the present invention provides a method for pretreatment of a biological sample comprising the above thawing step (2'). As the freezing temperature and freezing time in step (2'), the same conditions as mentioned above in paragraphs [0029] and [0030] may be used.

### (3) Solubilization step

This is a step of solubilizing a biological sample containing a protein to be subjected to an immunoassay, using a surfactant.

The solubilization in this step is not particularly limited in the timing of adding a surfactant, insofar as a protein, in particular a membrane protein, contained in a biological sample is solubilized. For example, the surfactant may be added when thrawing a biological sample or thereafter. If freezing and thawing do not affect the solubilizing action of the surfactant and the surfactant does not influence freezing and thawing effects, the surfactant may be added to the biological sample before freezing.

The surfactant used in the present invention is generally used for solubilizing a protein, particularly a membrane protein. For example, a nonionic surfactant that has an excellent protein-solubilizing effect and that does not affect immunoassay results can be preferably used. Examples of preferable nonionic surfactants include polyoxyethylene octylphenyl ether and polyoxyethylene sorbitan mono-fatty acid esters. Examples of preferable polyoxyethylene octylphenyl ethers include polyoxyethylene(8) octylphenyl ether (Triton X-114), polyoxyethylene(9) octylphenyl ether (NP-40), and polyoxyethylene(10) octylphenyl ether (Triton X-100). Polyoxyethylene(10) octylphenyl ether (Triton X-100) is more preferable.

Examples of preferable polyoxyethylene sorbitan mono-fatty acid esters include polyoxyethylene sorbitan monolaurates (in particular, polyoxyethylene(20) sorbitan monolaurate (Tween 20), polyoxyethylene sorbitan monopalmitates (in particular, polyoxyethylene(20) sorbitan monopalmitate (Tween 40)), polyoxyethylene sorbitan monostearates (in particular, polyoxyethylene(20) sorbitan monostearate (Tween 60)), and polyoxyethylene sorbitan monooleates (in particular, polyoxyethylene(20) sorbitan monooleate (Tween 80)). Among these, polyoxyethylene sorbitan monolaurates are preferable, and polyoxyethylene(20) sorbitan monolaurate (Tween 20) is particularly preferable.

All of these surfactants are commercially available, and can be purchased, for example, from Wako Pure Chemical Industries, Ltd., and Nacalai Tesque, Inc.

The amount of surfactant added to the biological sample containing a protein in the solubilization step may vary according to the type of surfactant used. For example, the surfactant may be added in an amount to achieve a surfactant concentration of 0.0001 to 30 wt.%, preferably 0.01 to 10 wt.%, and more preferably 0.1 to 2 wt.%, per 100 wt.% of the biological sample to be treated. In other words, the amount of surfactant is 0.01 to 30000 parts by weight, preferably 10 to 10000 parts by weight, and more preferably 100 to 2000 parts by weight per 100 parts by weight of the total amount of the protein contained in the biological sample.

The conditions for the solubilization step are not particularly limited insofar as the target protein, in particular membrane protein, can be dissolved without losing the immunological activity to be measured. However, the conditions are typically a pH of 4 to 10, preferably a pH of 6.5 to 8.5, and a temperature of 0 to 50°C, and preferably 4 to 37°C.

The biological sample obtained by being subjected to steps (1) to (3) or steps (2') and (3) may be subjected to the immunoassay described below as is or after being subjected to solid-liquid separation or any fractionation, if necessary.

### (II) Immunoassay of Protein Contained in the Biological Sample

The present invention relates to an immunoassay for determining a protein, particularly a membrane protein, contained in a biological sample pretreated in the above step (I). The immunoassay as used herein includes both of methods for the qualitative detection of a protein contained in a biological sample, and methods for the quantitative detection of a protein contained in a biological sample.

The immunoassay as used herein includes conventional enzyme immunoassay (EIA), radioimmunoassay (RIA), enzyme-linked immunosorbent assay (ELISA), agglutination, nephelometry, turbidimetry, and Western blot. These methods can be suitably selected according to the purpose. ELISA is preferable.

All of the above immunoassays are conventional methods known in the art, and can be easily performed according to (or pursuant to) textbook-like books and documents, such as Eiji ISHIKAWA, "Enzaimu imunoassei wa ko kaihatsu suru (This is How an Enzyme Immunoassay is Developed)", Gakkai shuppan senta, July, 2003. For the sake of convenience, commercially available products may be used as antibodies (polyclonal antibodies, monoclonal antibodies) used in such immunoassays. When not commercially available, such antibodies can be prepared according to (or pursuant to) textbook-like books and documents, such as "Tanpakushitsu jikken handobukku: bunri seisei shitsuryo bunseki kotai sakusei bunshikan sogo sayo kaiseki nado no kihon genri to saishin purotokoru soshuhen (Protein Experiment Handbook - Basic Principles and Current Protocols of Separation/Purification, Mass Spectrometry, Antibody Production, Molecular Interaction Analysis)", summary of the full version, Yodosha Co., Ltd., August, 2003).

A preferable embodiment of the present invention is an immunoassay that uses, as a target sample, a human-derived biological sample subjected to the pretreatment of the present invention, particularly urine, and that determines the amount of a protein contained in the biological sample, for example, a membrane protein such as aquaporin (AQP), preferably human AQP2, as shown in the Experimental Examples.

ELISA is preferably used as the immunoassay. For example, the antibody to AQP used in ELISA can be produced by a standard method, or with reference to Reference Production Examples 2 and 3 described below. The ELISA using urine as a test sample can be performed according to the protocol described in Experimental Example 1 described below. As described in Experimental Example 2, the pretreatment of the present invention enables highly accurate and efficient detection of the amount of a membrane protein, such as AQP, contained in urine.

### [Examples]

Hereinafter, a configuration of the present invention and effects thereof will be described based on Reference Production Examples and Experimental Examples. However, the present invention is not limited to any degree by such Experimental Examples and the like.

### Reference Production Example 1 Production of E. coli expressed Trx/His fused human AQP

Human aquaporin 2 (AQP2) gene was cloned and expressed in *E. coli.* Human AQP2 protein is a five-transmembrane protein that is composed of 271 amino acids, and whose protein domain has a molecular weight of about 30 kD (Sasaki S, et al., Cloning, characterization, and chromosomal mapping of human aquaporin of collecting duct. Clin. Invest.. 1994; 93(3): 1250-1256.). Here, human AQP2 protein was expressed in *E. coli* in a state of having its N-terminal tagged with Trx.

The method is summarized as follows.

First, the gene corresponding to human AQP2 (271 amino acids) was divided into two portions to be subjected PCR cloning using a first-half primer pair and a second-half primer pair. The PCR products were each incorporated in a plasmid, the plasmids were transformed into *E. coli,* and the nucleic acid sequences of those were determined. Then, clone 1 (first half portion) and clone 4 (second half portion) that had the same sequence as that of a wild type were selected, cleaved at an overlapping common restriction enzyme site (BamHI), and corresponding portions were ligated by a ligase to obtain a gene sequence. Then, the AQP2 gene sequence was inserted into a His-tag-fusion protein expression vector, to obtain an intended His-tag-fused AQP2 expression vector (clone 8). However, since it did not express the AQP2 protein in sufficient quantity, a NcoI-BamHI nucleic acid fragment lacking an N-terminal portion was cut out and incorporated in a Thioredoxin (Trx) fusion vector. The Trx-fused AQP2 expression vector (clones 2 and 8) was introduced into E. coli, and when induction was conducted using IPTG, it was found that a Trx-fused AQP2 protein was expressed in large quantity.

This human AQP protein was confirmed using an antibody produced from commercially-available synthetic human C-terminal peptides.

### (1) PCR amplification and cloning of human AQP2 gene

Human AQP2 gene was separately amplified as a first half portion (AQP2N: 86-749) and a second half portion (AQP2C: 562-901) from a human cDNA library (QUICK-Clone cDNA Human cDNA, CLONTECH), using primers specific thereto.

Specifically, as shown in FIG. 1, PCR of human AQP2 gene was performed by dividing the region (86-901) coding for human AQP2 protein (271 amino acids) at the HindIII site (position 609) into two portions, that is, an N-terminal portion and a C-terminal portion. As PCR primers for the human AQP2 gene N-terminal portion (hAQP2 / first half portion, referred to as "hAQP2N" hereinafter), 22 bases (86-107) from the start codon were used as a Forward primer (hAQP2N-F: SEQ ID NO. 2), and 22 bases of 728-749 were used as a Reverse primer (hAQP2N-R: SEQ ID NO. 3). On the other hand, with respect to the human AQP2 gene C-terminal portion (hAQP2 / second half portion, referred to as "hAQP2C" hereinafter), 22 bases of 562-583 were used as a Forward primer (hAQP2C-F: SEQ ID NO. 4), and 21 bases (881-901) up to the stop codon were used as a Reverse primer (hAQP2C-R: SEQ ID NO.5). To each of the 5' terminals of hAQP2N-F and hAQP2C-R, a restriction enzyme site (hAQP2N-F: SphI, hAQP2C-R: BamHI) to be used for insertion into the expression vector was added. FIG. 1 shows AQP2 gene and the configurations of the primers for the first half portion and the second half portion. As a result of PCR, PCR products of 884 bp (hAQP2N portion) and 340 bp (hAQP2C portion) that had been estimated were obtained.

The AQP2N (86-749) PCR product was incorporated into a pT7 cloning vector, to be transformed into *E. coli*, whereby *E. coli* colony Nos. 1, 2, 3, 5, and 6 were obtained. Plasmid DNAs were extracted from these *E. coli* colonies to be subjected to PCR using the specific first-half primers (NF/NR). As a result, it was confirmed that the intended AQP2N (86-749) sequence was inserted within the *E. coli* colony Nos. 1, 3, and 5. Further, two types of restriction enzymes were caused to act on the five types of plasmids to cut them into fragments. Then, it was confirmed that the nucleic acid fragments had sizes derived from AQP2 gene.

Similarly, the AQP2C (562-901) was also amplified, using the specific second-half primers, *E. coli* colony Nos. 1 to 6 were examined, and it was found that the gene was inserted within all the colonies. Plasmids from the colonies excluding No. 5, i.e., Nos. 1, 2, 3, 4, and 6, were extracted, and the sizes of the inserted genes were examined also using two restriction enzymes. The size of the nucleic acid fragment cut out by the restriction enzymes from the five types of plasmids was 2200 bp as had been estimated. Therefore, it was judged that the gene including AQP2C (562-901) was incorporated in the plasmids.

A total of 10 types of nucleic acid sequences, which are those of AQP2N (86-749) plasmid Nos. 1, 2, 3, 5, and 6, and AQP2C (562-901) plasmid Nos. 1, 2, 3, 4, and 6, were determined, using CEQ2000 of Beckman-Coulter. Then, the nucleic acid sequences of the plasmids were each compared to a corresponding wild type sequence (FIG. 2). As can be understood from FIG. 2, AQP2N (86-749) plasmid No. 1 and AQP2C (562-901) plasmid No. 4 which have the same sequence as that of a wild type were selected, and a determination was made to create a vector for expression by ligating fragments from both plasmids.

### (2) Construction of His-tag-fused human AQP2 expression vector

A SphI-BamHI fragment was cut out from the AQP2N (86-749) plasmid No. 1, and a BamHI-HindIII fragment was cut out from the AQP2C (562-901) plasmid No. 4. Then, the two fragments were incorporated, using a ligase, into a His-tag-fusion expression vector (pGL80L) whose ring was opened at SphI and HindIII sites. Colony PCR was performed using the F2/R primer pair, and it was confirmed that the fragments were inserted within *E. coli* colonies No. 2 and No. 8.

Plasmids were extracted from *E. coli* colonies No. 2 and No. 8, and were examined whether nucleic acid fragments having prescribed sizes (SphI-HindIII: 664 bp, SphI-BamHI: 514 bp, BamHI-HindIII: 340 bp) were present, using two types of restriction enzymes specific to AQP2 gene. As a result, the same nucleic acid fragments were found in No. 8, and it was confirmed that AQP2 gene was inserted therein. No. 8 was examined again to confirm the nucleic acid sequence, using CEQ2000 of Beckman-Coulter, and it was confirmed that there were no substitutions or deletions in the nucleic acid sequence.

### (3) Construction of Trx-fused N-terminal deficient AQP2 expression vector

Although the intended His-tag-fused AQP2 expression vector (clone 8) was obtained in (2) above, it did not express the AQP2 protein in sufficient quantity. Therefore, a NcoI-BamHI fragment lacking an N-terminal portion of human AQP2 protein was cut out from the His-tag-fused AQP2 expression plasmid No. 8, and incorporated, using a ligase, into a Trx-fused expression vector (pET32b) whose ring was opened at NcoI and HindIII sites. Colony PCR was performed using the F2/R primer pair, and it was confirmed that the fragment was inserted within E. *coli* colonies No. 2 and No. 8.

Plasmids were extracted from *E. coli* colonies No. 2 and No. 8, and were examined whether a nucleic acid fragment having a prescribed size (NcoI-HindIII: 686 bp) was present, using two types of restriction enzymes specific to AQP2 gene. It was confirmed that a 686 bp nucleic acid fragment existed in No. 2 and No. 8 and an N-terminal portion lacking AQP2 gene was inserted therein.

### (4) Expression of Trx-fused N-terminal deficient AQP2 protein in E. coli

Since there were no errors in the sequences of Trx-fused N-terminal deficient AQP2 expression vectors No. 2 and No. 8, *E. coli* of each colony was precultured, added to a 100 mL LB medium, and induced overnight at room temperature using IPTG (Isopropyl-b-D-thiogalactopyranoside: a b-galactosidase inducer which is used as an expression inducing agent for an expression vector having a Lac promoter or a tac promoter), whereby a Trx-fused N-terminal deficient AQP2 protein was produced in *E. coli* cells.

Two types (No. 2 and No. 8) of *E. coli* were cultured. Pre-IPTG samples (before induction), post-IPTG samples (after induction), and insoluble fractions and soluble fractions which were obtained by solubilizing the induced *E. coli* were prepared for SDS-PAGE/CBB staining and Western analysis. For detection, a commercially-available synthetic human AQP2 peptide antibody (anti-human AQP2 rabbit serum, PherMingen) was used. As can be seen in FIG. 3, expression of the Trx-fused N-terminal deficient AQP2 protein was not confirmed in the pre-IPTG induction samples 2) and 7). However, a large amount of the Trx-fused N-terminal deficient AQP2 protein was confirmed in the post-IPTG induction samples 3) and 8). Since the fusion protein existed more in the *E. coli* insoluble fractions 4) and 9) than in the soluble fractions 5) and 10), the fusion protein was found to exist as an inclusion body.

As described above, human AQP2 gene was cloned from human cDNA by PCR, and it was confirmed that the obtained human AQP2 gene completely matched that of the wild type. Further, by using this human AQP2 gene, it was possible to express the Trx-fused human AQP2 protein lacking about 30 amino acids of the N-terminal of human AQP2 protein.

Hereinafter, this protein will be referred to as "*E. coli* expressed Trx/His fused human AQP2 protein" or "Trx-fused human AQP2 protein".

### Reference Production Example 2 Production of His-tagged human AQP2 expressed in insect cell Sf-9 (hAQP2/His/Sf-9) See FIG. 4 (1))

A cDNA with a His-tagged sequence added to the 5' terminal of AQP2 was inserted to a multicloning site of donor plasmid pFastBacl (pFastBacl-AQP2). *E. coli* DH10Bac competent cells were transformed using the pFastBacl-AQP2. With respect to clones that were white on an agar plate having added therein Bluo-gal and IPTG, confirmation was conducted by PCR whether a Bacmid includes insertion of AQP2 (Recombinant AQP2 Bacmid). The Recombinant AQP2 Bacmid was extracted, purified, and transfected to Sf-9 cells, using CELLFECTIN (Invitrogen). The culture supernatant after 72 hours from the transfection was collected, to be used as a Recombinant Baculovirus (rBaculovirus-AQP2). Until a sufficient titer of the rBaculovirus-AQP2 was obtained in the culture supernatant, infection, amplification, collection, and re-infection of the rBaculovirus-AQP2 to Sf-9 cells were repeated. The Sf-9 cells were infected with the rBaculovirus-AQP2, cultured for 2 to 4 days, and the cells were collected. SDS-PAGE was performed using the cells, and expression of the rBaculovirus-AQP2 was confirmed by Western blotting using the AQP2 antibody. FIG. 4 (2) shows the results.

### Reference Production Example 3 Rabbit polyclonal antibody against human AQP2

The Trx-fused human AQP2 protein produced in Reference Production Example 1 was purified and used for immunizing rabbits (species/strain: Rabbit/New Zealand White, sex: female, weight: 3 to 3.5 kg, obtained from KITAYAMA LABES), to produce a polyclonal antibody.

### (1) Preparation of immunogen

Used as an immunogen was the Trx-fused human AQP2 protein (hAQP2/Trx) purified, using a Ni-NTA agarose gel, from the *E. coli* in which expression of human AQP2 had been confirmed in Reference Production Example 1. For the immunization, the immunogen (hAQP2/Trx) was prepared to be 200 µg/mL, using PBS(-). 2.5 mL of the immunogen was mixed with an equal volume of Complete Freund adjuvant (DIFCO, Cat No. 263810), using two glass syringes connected by a three-way stopcock, to prepare an emulsion.

### (2) Preparation of polyclonal antibody

### (2-1) Immunization

The immunogen (hAQP2/Trx) prepared above was intradermally injected on the back of rabbits every 2 weeks at multiple points by 1 mL/body for immunization. After 1 week from the third immunization, partial blood collection was performed from an earlobe, and the antibody titer was checked by the ELISA method described in (2-2) below. Further, additional immunizations were performed, and after 1 week from the seventh immunization, whole blood collection was performed from the carotid artery. The collected blood was centrifuged at 3,000 rpm for 10 minutes at room temperature to collect the serum. The antibody titer of the antiserum was checked by the ELISA method described in (2-2) below, and the reactivity in Western blotting was examined.

### (2-2) Measurement of antibody titer by ELISA

The immunogen (hAQP2/Trx) prepared to be 1 µg/mL using PBS(-) was put into a 96 well plate by 100 µL/well, to be left still at room temperature for 2 hours or more. On the other hand, with respect to the hAQP2/His expressing Sf-9 cells (hAQP2/His/Sf-9) (Reference Production Example 2), the hAQP2/His expressing Sf-9 cells (hAQP2/His/Sf-9) that had been dissolved in 8M Urea/50 mM Tris-HC1 (pH8.0) were plated in a 96 well plate by 10 µL/well, PBS(-) was added thereto by 100 µL/well, and the plate was left still at room temperature for 2 hours or more.

The immunogen solution was removed from the plate, 0.1% BSA/PBS(-) was added by 400 µL/well to the plate, and blocking was conducted at room temperature for 1 hour or more (immunogen coated plate and hAQP2/His/Sf-9 coated plate).

The antiserum obtained through partial or whole blood collection was diluted by 1,000-fold using 0.1% BSA/PBS(-), and was subjected to eight serial dilutions at a common ratio of 3 (dilution series: 1,000-fold to 2,187,000-fold). As a control serum, Normal Rabbit Serum (NRS) was diluted and prepared in the same manner (dilution series: 1,000-fold to 2,187,000-fold).

The blocking solution was removed from the 96 well plate, and the dilution series of the antiserum or the NRS was added by 100 µL/well, and the mixture was allowed to react overnight at room temperature.

Next day, the 96 well plate was washed three times with a washing solution (0.05% Tween-20/0.9% NaCl), Horseradish peroxidase (HRP)-goat anti rabbit IgG (Biosource, Cat. No. ALI3404) diluted 10,000-fold using 0.05% Tween-20/0.1% BSA/PBS(-) was added by 100 µL/well, and the mixture was allowed to react at room temperature for 2 hours. Next, the plate was washed five times with a washing solution. Then, a coloring solution obtained by dissolving OPD tablets (Sigma, Cat. No. P8287) in a substrate dilution solution (citric acid monohydrate 7 g/L, Na₂HPO₄·12H₂O 23.89 g/L, 0.015% H₂O₂) at 1 mg/mL was added by 100 µL/well to allow coloring to proceed at room temperature for 5 to 10 minutes. 2 N H₂SO₄ was added by 100 µL/well to stop the reaction, and the absorbance at 492 nm was measured using an iEMS Reader MF (Lab systems).

### (2-3) Examination of reactivity by Western blotting

The immunogen (hAQP2/Trx) and a Trx/His control (obtained by substituting an AQP2 portion with a C-terminal portion 743-968 of PKD2 protein, that is, PKD2 with a Trx/His tag added thereto) were diluted to 10 µg/mL with a 2-mercaptoethanol containing lysis buffer for electrophoresis, and the mixtures were heated at 100°C for 5 minutes. The antigen solutions and a molecular weight marker (Bio-Rad, Cat. No. 161-0373) were applied to a 15% acrylic amide gel by 10 µL/well, and the samples were analyzed by electrophoresis at 25 mA/gel.

After the electrophoresis, one gel was stained by CBB G-250 stain (Bio-Rad Cat. No. 161-0786), and the proteins in another gel were transferred to a 0.45 µm nitrocellulose membrane (Bio-Rad, Cat. No. 162-0115) at 200 mA for 1 hour, using a semidry blotter. After the transfer, the membrane was immersed in a 5% skim milk / 50 mM Tris-HCl (pH 8.0) at room temperature for 1 hour or more to be blocked. The antiserum and the NRS were each diluted 1,000-fold using 0.1% BSA/PBS(-), and were allowed to react with a membrane overnight at room temperature.

Next day, the membrane was washed with a washing solution, and was allowed to react with HRP-goat anti rabbit IgG (Biosource, Cat. No. ALI3404) diluted 3,000-fold using 0.05% Tween-20/0.1% BSA/PBS(-), at room temperature for 2 hours. The membrane was washed with a washing solution and was immersed in a coloring solution for coloring. The coloring solution was prepared by dissolving 4.84 g of tris (hydroxymethyl) aminomethane, 5.84 g of sodium chloride, and 3.36 mL of concentrated hydrochloric acid in H₂O to obtain a total volume of 1 L. For usage, 20 mL of 0.3% 4-chloro-1-naphtohl/methanol and 60 µL of 30% H₂O₂ were added to 100 mL of this coloring solution, and mixed.

### (2-4) Results of polyclonal antibody preparation

FIG. 5 shows results of the ELISA performed on the dilution series (1,000-fold to 2,187,000-fold) of the sera collected through the partial blood collection performed after the third immunization, using a 96 well plate on which a solid phase of hAQP2/Trx or the Sf-9 cell expressed hAQP2 (hAQP2/Trx/Sf-9) had been formed. In FIG. 5, (A) shows antibody titers against the immunogen (hAQP2/Trx), and (B) shows antibody titers against the Sf-9 cell expressed hAQP2 (hAQP2/Trx/Sf-9). As seen from the results, it was confirmed that the antibody titer against the immunogen was sufficiently increased through three immunizations to a rabbit.

FIG. 6 shows the results of ELISA assays performed in a similar manner after the whole blood collection performed after the seventh immunization. In FIG. 6, (A) shows antibody titers against the immunogen (hAQP2/Trx), and (B) shows antibody titers against the Sf-9 cell expressed hAQP2 (hAQP2/Trx/Sf-9). The respective polyclonal antibodies obtained at this time were named OPP21401, OPP21402, OPP21403, and OPP21404. The volumes of the antisera were 50 mL, 52 mL, 50 mL, and 68 mL, respectively. Further, final antibody titers were confirmed by ELISA, and it was found that OPP21403 showed the highest antibody titer.

Further, the reactivity of each polyclonal antibody against each type of antigen (hAQP2/Trx, hAQP2/Trx/Sf-9, and Trx/His control) was examined by Western blotting. Specifically, each antigen (hAQP2/Trx, hAQP2/Trx/Sf-9, and Trx/His control) transferred to a nitrocellulose membrane was allowed to react with each antiserum diluted 1,000-fold for coloring, using a 4-chloro-1-naphthol method. FIG. 7 shows the results. As shown in FIG. 7, all antibodies (OPP21401, OPP21402, OPP21403, and OPP21404) reacted not only to hAQP2/Trx but also hAQP2/His/Sf-9. OPP21403 had the strongest reaction with hAQP2/His/Sf-9.

From this, among the four types of polyclonal antibodies obtained above, it was considered that OPP21403, which had been expressed in Sf-9 cells and which had the strongest reaction with hAQP2, was appropriate for detection of AQP2 in a living body-derived sample.

**[Table 1]**

| Antibody name | Immunogen | Immunized animal | Form | Reactivity | | |
|---|---|---|---|---|---|---|
| | | | | hAQP2/ Trx | hAQP2/ His/Sf-9 | Trx/His control |
| OPP21401 | *E. coli* expressed Trx/His fused human AQP2 | Rabbit | Serum | + | + | - |
| OPP21402 | *E. coli* expressed Trx/His fused human AQP2 | Rabbit | Serum | + | + | - |
| OPP21403 | *E. coli* expressed Trx/His fused human AQP2 | Rabbit | Serum | + | + | - |
| OPP21404 | *E. coli* expressed Trx/His fused human AQP2 | Rabbit | Serum | + | + | - |

### Reference Production Example 4 Mouse monoclonal antibody against human AQP2

The Trx-fused human AQP2 protein (hAQP2/Trx) produced in Reference Production Example 1 was purified and intraabdominally injected for immunization to mice (species/strain: Mouse/Balb/c, sex: female and male, obtained from SLC), and a monoclonal antibody was produced through cell fusion with mouse myeloma cells (cell line name: P3U1, obtained from ATCC).

### (1) Preparation of monoclonal antibody

### (1-1) Immunization

1 mL of the immunogen (hAQP2/Trx) prepared in Reference Production Example 3 (1) was intraabdominally injected for immunization to ten mice (species/strain: Mouse/Balb/c, sex: female and male, obtained from SLC) three times every 2 weeks. 3 days before cell fusion, hAQP2 was injected by about 20 µg/body as the last immunization without using an adjuvant.

### (1-2) Cell fusion

Mouse myeloma cells (cell line name: P3U1, obtained from ATCC) were thawed at least 1 week before cell fusion, and cultured in 10% FCS/RPMI 1640. On the day of the cell fusion, it was confirmed under a microscope that the mouse myeloma cells had sufficient viability.

A single mouse (female) that had been immunized in (1-1) above was anesthetized using ether, disinfected using 70% ethanol, and then had its spleen sterilely taken out. The spleen was finely chopped with scissors, and was passed through a steel-mesh in about 10 mL of RPMI 1640 to be strained. This suspension was transferred to a 15 mL centrifugation tube and was centrifuged. Then, the supernatant is discarded, and the precipitate was loosened thoroughly through tapping with a finger. 4 mL of 0.747% NH₄Cl was added to the precipitate, and unnecessary red blood cells were hemolyzed by slightly pipetting the mixture. Then, 6 to 7 mL of RPMI 1640 was added thereto, and the mixture was centrifuged. Then, the cells were washed with RPMI 1640 again, and re-suspended in about 10 mL of RPMI 1640.

On the other hand, 50 to 100 mL of the P3U1 culture solution was transferred to a 50 mL centrifugation tube, and subjected to centrifugation to collect cells. The cells were washed once with RPMI 1640, and then re-suspended in RPMI 1640. The suspension of the spleen cells and the suspension of P3U1 were combined to be a volume of about 30 mL, and the mixture was centrifuged. The supernatant was discarded and the precipitate was loosened thoroughly through tapping with a finger. 2 mL of 50% PEG Solution (Sigma, Cat. No. P7181) was added to the precipitate over 30 seconds, while agitating the mixture well, and the mixture was further pipetted for 30 seconds. Then, 5 mL of RPMI 1640 was added to the mixture over 2 minutes, 5 mL of RPMI 1640 was further added thereto, the mixture was left still at 37°C for 3 minutes, and then the mixture was centrifuged.

The supernatant was discarded and the cells were re-suspended in about 100 mL of HAT (ICN, Cat. No. 1680849) added 10% FCS/RPMI 1640, and the total amount of the cells was plated in four 24 well culture plates to be cultured at 37°C in 5% CO₂ (CO₂ incubator; SANYO, MCO-17AIC). The centrifugation condition was room temperature, 1200 rpm, and 5 minutes, for all cases.

### (1-3) Antibody screening

After 1 week to 10 days from the cell fusion at the time point when it became possible to visibly confirm formed colonies from the bottom of each plate, a portion of the culture supernatant was collected from each well to perform an antibody screening. In the first screening, an undiluted solution of the culture supernatant was used, and in the second screening and thereafter, the culture supernatant was diluted 2.5-fold using 0.1% BSA/PBS(-) to be used.

The blocking solution was removed from the immunogen coated plate and the hAQP2/His/Sf-9 coated plate produced in a manner similar to that described in Reference Production Example 3 (2) (2-2), the culture supernatant was added to each plate by 100 µL/well, and the mixture was allowed to react overnight at room temperature.

Next day, each of the plates was washed three times using a washing solution, HRP-goat anti mouse IgG (Bio-Rad, Cat. No. 170-6516) that had been diluted 5,000-fold using 0.05% Tween-20/0.1% BSA/PBS(-) was added to the plate in an amount of 100 µL/well, and the mixture was allowed to react at room temperature for 2 hours. Then, the plate was washed five times with a washing solution. An OPD tablet-dissolved coloring solution, which had been prepared in a similar manner to that described in Reference Production Example 3 (2) (2-2), was added to the plate by 100 µL/well, to allow coloring to proceed for 10 to 30 minutes at room temperature. 100 µL/well of 2 N H₂SO₄ was added to stop the reaction. Then, the absorbance at 492 nm was measured using the iEMS Reader MF (Lab systems).

Wells that were positive both in the immunogen coated plate and the hAQP2/His/Sf-9 coated plate were selected for cloning.

### (1-4) Hybridoma cloning

Hybridoma cloning was performed by a limiting dilution method.

Immune mice (male) anesthetized with ether were disinfected using 70% ethanol, and then had their spleens sterilely taken out. Feeder cells were prepared through similar operations as in the spleen cell preparing method described in (1-2) above. Spleen cells of a single mouse were re-suspended in about 40 mL of 10% FCS/RPMI 1640, and were seeded on four 96 well culture plates in an amount of 100 µL/well. Cells in the positive wells in (1-3) above were serially diluted in accordance with the number of cells, and were seeded in an amount of 100 µL/well onto a 96 well culture plate on which the feeder cells had been seeded, and the cells were cultured at 37°C in 5% CO₂.

Cloning and screening were repeated several times, and wells that were positive in the screening and were confirmed to contain a single clone through microscopic observation were selected. This clone was cloned again by the limiting dilution method. Then, in the next screening, it was confirmed that all wells in which cells existed were positive and wells in which cells did not exist were negative. A single clone was selected from this plate and was established as a monoclonal antibody producing hybridoma.

The established hybridoma was serially cultured to expand in 10% FBS/RPMI 1640 to make about 50 mL of a cell culture solution (hybridoma culture solution). About 40 mL of this solution was transferred to a 50 mL centrifugation tube, and centrifuged at room temperature at 1200 rpm for 5 minutes to collect the cells (hybridoma). The cells were suspended in 3 mL of a cell bunker (JUJI FIELD, Cat. No. BLC-1), and the suspension was dispensed into three cryotubes by 1 mL and preserved in liquid nitrogen. A portion of the centrifuged supernatant was preserved at 4°C and subjected to antibody typing and Western blotting.

### (1-5) Ascitic fluid collection

In order to obtain a purified antibody to be used for establishing an ELISA assay system, the hybridoma obtained above was injected into mice and ascites containing large amount of antibodies was collected.

Specifically, about 10 mL of the cell culture solution (hybridoma culture solution) prepared in (3-4) was centrifuged at room temperature at 1200 rpm for 5 minutes, to collect the cells (hybridoma). The cells washed once with PBS(-) were re-suspended in 1.5 mL of PBS(-), and 0.5 mL aliquots of the suspension were injected into the peritoneal cavities of three male mice. Each of the mice had been intraperitoneally injected in advance with pristine (2, 6, 10, 14-tetramethylpentadecan; Sigma, Cat. No. T7640) by 0.5 mL/body at least 3 days before the cell injection.

Ascites were collected when the sizes of the abdomens of the mice injected with the cells were at a maximum. Ascites of a single mouse was taken sterilely, and was centrifuged at room temperature at 1200 rpm for 5 minutes to collect a supernatant. Then, red blood cells in the precipitate were hemolyzed using 0.747% NH₄Cl, as in the feeder cell preparation. The cells were washed with RPMI 1640, and then suspended in 3 mL of a cell bunker. The suspension was dispensed into three cryotubes by 1 mL, to be preserved in liquid nitrogen. Ascites of the other two mice were centrifuged at room temperature at 3000 rpm for 10 minutes to collect supernatants. The supernatants derived from the three mice were preserved at 4°C.

### (1-6) Antibody typing

The obtained antibodies were classified into subclass and L chain types, using anti-mouse Ig (IgG1, G2a, G2b, G3, M, k chain, and 1 chain) (BIONETICS) antisera.

The blocking solution was removed from an immunogen coated plate produced in a similar manner to that in Reference Production Example 3 (2) (2-2), the culture supernatant obtained in (1-4) was added thereto in an amount of 100 µL/well and allowed to react overnight at room temperature. Next day, the plate was washed three times with a washing solution. Each of the anti-mouse Ig (IgG1, G2a, G2b, G3, M, k chain, and 1 chain) antisera was diluted 1,000-fold using 0.1% BSA/PBS(-), and 100 µL of each of the dilutions was added to a well. The mixture was allowed to react at room temperature for 2 hours, the plate was washed three times with a washing solution, HRP-goat anti rabbit IgG diluted 10,000-fold using 0.05% Tween-20/0.1% BSA/PBS(-) was added by 100 µL/well, and the mixture was allowed to react at room temperature for 2 hours. Next, the plate was washed five times with a washing solution, an OPD tablet-dissolved coloring solution that had been prepared in a similar manner to that in Reference Production Example 3 (2) (2-2) was added thereto in an amount of 100 µL/well, to allow coloring to proceed at room temperature for 10 minutes. 100 µL/well of 2 N H₂SO₄ was added to stop the reaction, and the absorbance at 492 nm was measured using the iEMS Reader MF (Lab systems).

Those that exhibited the strangest reactions were determined as their subclass and L chain of the antibody.

### (1-7) Examination of specificity of antibody by Western blotting

Western blotting was performed in a similar manner to that in Reference Production Example 3 (2) (2-3), and blocked membranes were allowed to react overnight with the culture supernatant obtained in (1-4) at room temperature. Next day, each of the membranes was washed with a washing solution, and was allowed to react with HRP-goat anti mouse IgG diluted 3,000-fold using 0.05% Tween-20/0.1% BSA/PBS(-), at room temperature for 2 hours. The membrane was washed with a washing solution, and coloring was allowed to proceed using a similar method to that described in Reference Production Example 3 (2) (2-2).

### (2) Result of monoclonal antibody preparation

Through the above operations, two clones of the monoclonal antibody producing hybridoma were established, from ten hAQP2/Trx immune mice. These hybridomas were named OPH21401 and OPH21402, and antibodies produced from the hybridomas were named OPM21401 and OPM21402, respectively. For each of OPM21401 and OPM21402, the subclass was IgGl and L chain was *K* chain.

The specificities of these antibodies were examined by Western blotting. As shown in FIG. 8, both antibodies reacted with both of hAQP2/Trx and hAQP2/His/Sf-9, and did not react with a Trx/His control. Therefore, it is considered that the two types of monoclonal antibodies obtained above are antibodies specific to human AQP2.

**[Table 2]**

| Antibody name | Immunogen | Immunized animal | Form | Reactivity | | |
|---|---|---|---|---|---|---|
| | | | | hAQP2/ Trx | hAQP2/His/ Sf-9 | Trx/His control |
| OPM21401 | *E. coli* expressed Trx/His fused human AQP2 | Mouse | Ascites | + | + | - |
| OPM21402 | *E. coli* expressed Trx/His fused human AQP2 | Mouse | Ascites | + | + | - |

### (3) Purification of monoclonal antibody

4 mL of a Binding Buffer (3.3 M NaCl/1.5 M glycine, NaOH 8.5 g/L) was added to 2 mL of the mouse ascites collected in (1) (1-5) above, and the mixture was left still at room temperature for 2 hours, and then centrifuged under a condition of 3,000 rpm, 30 minutes, and 4°C to collect a supernatant. This supernatant was filtered with a 0.45 µm filter, and applied to a Protein-A Sepharose column which had been equilibrated in advance with the above Binding Buffer. The column was washed with the Binding Buffer, eluted with an Elution Buffer (Bio-Rad, Cat. No. 153-6162), and immediately neutralized with 2 M Tris. The eluate was dialyzed against 0.1 M NaHCO3, then its absorbance at a wavelength of 280 nm was measured to obtain an IgG concentration using the calculation formula below.

IgG concentration (mg/mL) = OD₂₈₀ value of IgG solution x 0.714.

### Experimental Example 1 Establishment of aquaporin 2 (AQP2) ELISA

In order to promptly monitor pathologic conditions of polycystic kidney disease (PKD), it was examined whether aquaporin 2 (AQP2) can be used as one candidate protein of biomarkers for the disease, whether an assay system (ELISA) using an antibody against aquaporin 2 (AQP2) can be established, and whether PKD pathologic conditions can be actually monitored using the assay system.

For the ELISA, a polyclonal antibody (antibody name: OPP21403) (see Reference Production Example 3) and a monoclonal antibody (antibody name: OPM21401) (see Reference Production Example 4) were used which had been respectively produced by: cloning the human AQP2 gene, adding a Trx tag to the N-terminal thereof, producing "Trx-fused AQP2 protein" (see Reference Production Example 1) by expressing the Trx-tagged gene in *E. coli*, and immunizing rabbits and mice with the produced protein.

As a standard protein for the ELISA, a "GST-fused hAQP2 protein" expressed in *E. coli* was used. As a control, the His-tagged human AQP2 protein "hAQP2-His/Sf-9" (see Reference Production Example 2) expressed in insect cells Sf-9 was used.

### (1) Tests for establishing the ELISA

### (1-1) Examination of test sample processing condition

Since AQP2 is a membrane protein buried in a membrane, conditions for solubilizing AQP2 from the membrane using various solubilizers (Urea, Triton X-100, NP-40, Tween-20, guanidine hydrochloride, and SDS), which do not affect the assay system, were examined.

Specifically, a liquid (hAQP2-His/Sf-9 suspension) obtained by suspending, in PBS, "hAQP2-His/Sf-9", which is a human AQP2 protein buried in a membrane of an insect cell, was mixed with each of the various solubilizers (Urea, Triton X-100, NP-40, Tween-20, guanidine hydrochloride, or SDS) in equal volumes, the mixture was diluted 10-fold using 10% FBS/0.1% BSA/PBS, and an ELISA was performed. Then, the coloring was measured as the absorbance at a wavelength of 492 nm.

FIG. 9 shows the results.

As can be understood from the results, it was confirmed that, compared to using Urea or guanidine hydrochloride, AQP2 could be solubilized from the membrane more efficiently and at a higher yield when, as a solubilizer, a surfactant such as Triton X-100, NP-40, or Tween-20 (which are nonionic surfactants), or sodium dodecyl sulfate (SDS, which is an anionic surfactant) was used.

### (1-2) Examination of stabilizer in ELISA assay system

In order to be able to stably measure the solubilized subject protein (AQP2) by ELISA, examination was conducted regarding a first buffer that is to be used for the reaction using a primary antibody.

Specifically, whether a stabilizer to be added to the first buffer (0.2% BSA / 10 mM EDTA / 0.2M Tris-HCl (pH 8.0)) affects the coloring in the ELISA assay system was examined by: adding each stabilizer (Urea, Triton X-100, NP-40, Tween-20, guanidine hydrochloride, and SDS) to the first buffer at various concentrations (dilution ratio relative to 1% concentration: 1-fold, 1/2-fold, 1/4-fold, 1/8-fold, 1/16-fold, 1/32-fold, 1/64-fold, and 1/128-fold); and measuring the absorbance of the subject protein (AQP2) at a wavelength of 492 nm.

The subject protein was prepared by: completely solubilizing a suspension of the hAQP2/Sf-9, which is a human AQP2 protein buried in a membrane of an insect cell, with the above-described solubilizer SDS; and diluting this mixture 2,000-fold using 0.1% BSA/PBS to eliminate the influence of SDS.

FIG. 10 shows the results. As shown in FIG. 10, when used as a stabilizer added to the first buffer, nonionic surfactants such as Triton X-100, NP-40, and Tween-20 increased the measurement sensitivity at concentrations equal to or higher than 0.063% (dilution ratio: 1/16), whereas Urea, guanidine hydrochloride, and SDS deteriorated the coloring. Thus, it was determined that 1% Triton X-100 is favorable as the stabilizer to be added to the first buffer; in other words, it was determined that 0.2% BSA / 10 mM EDTA / 2% Triton X-100 / 0.2M Tris-HCl (pH 8.0) is favorable as the first buffer to be used in the ELISA assay.

### (1-3) Human AQP2 assay system by Sandwich ELISA

In (1-4) to (1-6) described below, the following ELISA assay system was used in ELISA standard protein examination, test sample examination, and comparison examination with Western blotting.

### <Human AQP2 ELISA assay system>

100 µL of a monoclonal antibody (first antibody: OPM21403) prepared at a concentration of 5 µg/mL with 0.1 M NaHCO₃ was added to each well in a 96 well microplate (NUNC), and the plate was lidded and left still at room temperature for 2 hours. The first antibody solution was removed from the plate, and a blocking solution (0.1% BSA/PBS(-)) was added by 400 µL/well, and blocking was performed at room temperature for 1 hour or more. Then, the blocking solution was removed from the plate on which the first antibody was formed as a solid phase, 100 µL of the first buffer (0.2% BSA/2% Triton (registered trademark) X-100 / 10 mM EDTA / 200 mM Tris-HCl (pH8.0)) was added to each well, then, 50 µL of a standard protein or a test sample prepared to be respective concentrations using 0.1% BSA/PBS(-) was added thereto, and the plate was sealed and shaken overnight at room temperature.

Next day, after the plate was washed three times with a washing solution (0.05% Tween-20/0.9% NaCl), 100 µL of a polyclonal antibody (second antibody: OPP21403) diluted 5000-fold using 10% porcine serum/0.05% Tween-20/0.1 M Tris-HCl (pH8.0) was added to each well, and the plate was lidded and shaken at room temperature. After 2 hours of reaction, the plate was washed three times with a washing solution, then, 100 µL of Horseradish peroxidase (HRP)-goat anti rabbit IgG (Biosource, Cat. No. ALI3404) that had been diluted 10,000-fold using 0.05% Tween-20/0.1% BSA/PBS(-) was added to each well of the plate, and the plate was lidded and shaken at room temperature. After 2 hours, the plate was washed five times with a washing solution, then, 100 µL of TMB coloring solution (ScyTek, Cat. No. TM4999) was added to each well to allow coloring to proceed at room temperature. After 10 minutes, 100 µL of TMB Stop Buffer (ScyTek, Cat. No. TSB999) was added to each well of the plate to stop the reaction. Smudges and water on the bottom of the plate were cleaned off using a soft paper towel, and then the absorbances of respective wells at a wavelength of 450 nm were measured using a microplate absorptiometer (iEMS Reader MF, LABSYSTEMS).

A standard curve was generated based on the absorbance values of the standard protein, and the concentration of AQP2 contained in the test sample was determined based on the standard curve.

### (1-4) Examination of standard protein

Regarding the standard protein, by using the ELISA assay system described in (1-3), it was evaluated which of the *E. coli* derived AQP2 or the insect cell derived AQP2 is appropriate as the standard protein for ELISA assays.

As the *E. coli* derived AQP2, the "Trx-fused AQP2 protein" produced by the method described in Reference Production Example 1 was used. As the insect cell derived AQP2, the "AQP2-His/Sf9" produced by the method described in Reference Production Example 2 and solubilized by the solubilizer was used.

FIG. 11 shows the standard curves generated by performing ELISA assays described in (1-3) above, using the proteins above as the standard protein. As shown in FIG. 11, in both of the cases where the "Trx-fused AQP2 protein" was used as the standard protein and where the solubilized "AQP2/Sf9" was used as the standard protein, a concentration (dose)-dependent correlation with the measured absorbance was observed, and both curves extend parallel to each other. Thus, it was found that either protein can be used as the standard protein for the human AQP2 ELISA assay.

### (1-5) Examination of test samples

As test samples, four urine samples (Nos. 4, 5, 6, and 9) that had been collected from healthy human volunteers and had been serially diluted were used to perform the ELISA assays described in (1-3) above. FIG. 12 shows the results thereof, plotted along with a standard curve generated using the "Trx-fused AQP2 protein" as the standard protein.

As shown in FIG. 12, in all of the four urine samples used as test samples, a correlation similar to that in the standard curve was observed between the dilution ratio and the measured absorbance. Thus, it was confirmed that, using urine as the test sample, the above ELISA assay system allows detection and quantification of AQP2 contained in urine. Moreover, human AQP2 measurements were performed on the urine of ten healthy human volunteers in a similar manner. The concentrations of human AQP2 in the urine ranged from 0.93 to 8.84 ng/mL. Further, it was confirmed the detection limit in the above ELISA assay system was 0.01 ng/mL.

(A) of FIG. 13 shows results of measurements performed on urine of ten healthy human volunteers for quantifying human AQP2 in the urine by Western blotting using a monoclonal antibody (OPM21401) (Reference Production Example 3) against human AQP2. (B) of FIG. 13 shows the quantities of human AQP2 in the urine measured by the above ELISA assay system for the same ten healthy human volunteers. From these results, it was confirmed that the quantities of human AQP2 in the urine measured by the ELISA assay system of the present invention substantially correlate to the Western blotting results. It should be noted that, from the Western blotting results, it was found that the human derived AQP2 includes an AQP2 with a carbohydrate chain added thereto, and an AQP2 without a carbohydrate chain.

### (2) AQP2 ELISA protocol

Based on the examinations above, an AQP2 ELISA method having the following protocol was established.
1. Dilute an anti-AQP2 antibody (monoclonal antibody: OPM21401) to be 5 µg/mL using 0.1M NaHCO₃, dispense 100 µL of the dilution into each well, and leave the mixture still at room temperature for at least 2 hours.
2. Remove the anti-AQP2 antibody solution from each well, add 350 µL of a blocking solution (0.1% BSA/PBS(-)) to each well, and keep the plate at 4°C until use.
3. Remove the blocking solution from the plate on which a solid phase of the antibody has been formed, and add 100 µL of a first buffer (0.2% BSA / 10 mM EDTA / 2% Triton X-100 / 0.2M Tris-HCl (pH 8.0)) to each well.
4. To prescribed wells, add 50 µl of a human AQP2 reference standard at each of eight concentration levels (0, 0.01, 0.04, 0.12, 0.37, 1.1, 3.3, and 10 ng/ml) and 50 µl of a test sample (preferably, urine) that is to be measured, and shake the plate at room temperature for at least 2 hours.
5. Wash the plate, and add 100 µl of an anti-AQP2 antibody (polyclonal antibody: OPP21403) solution (diluted 5000-fold) to each well, and shake the plate at room temperature for 2 hours.
6. Wash the plate, add 100 µl of a solution obtained by diluting HRP-labeled anti-rabbit IgG 10,000-fold to each well, and allow reaction to proceed at room temperature for 2 hours.
7. Wash the plate, add 100 µL of a TMB solution to each well, allow reaction to proceed for 10 minutes, and then add 2N sulfuric acid to each well to stop the reaction.
8. Obtain an OD450 value using an absorption spectrometer, and determine an AQP2 concentration in the test sample based on a standard calibration curve (see FIG. 14) that has been generated based on the human AQP2 reference standard.

### Experimental Example 2 Examination (No. 1) of pretreatment conditions for the test sample

In order to examine pretreatment conditions for the test sample to be subjected to an immunoassay (ELISA) of human AQP2 protein established in Experimental Example 1, the following experiments were performed, using urine as the test sample. As the urine, fresh urine collected from one healthy human volunteer was used.

### (1) The first test and its results

The fresh urine was dispensed by 5 ml into five 10 mL tubes, and the tubes were each preserved for 24 hours, under conditions of 37° C, 23° C, 4° C, -30° C, and -80° C (in the dark). After the preservation, each sample was left still at room temperature to allow the xample to return to room temperature.

Using each sample as the test sample, the AQP2 ELISA established in Experimental Example 1 was performed to measure the amount of human AQP2 in the urine. As a control, the above AQP2 ELISA was performed on a portion of the fresh urine, immediately after the collection thereof without preserving it, to measure the amount of human AQP2 in the urine.

The amount of human AQP2 obtained from the control sample was defined as 100, and a relative ratio of the amount of human AQP2 obtained for each preserved sample was determined.

As a result, as shown in FIG. 15, the quantities of human AQP2 in the urine preserved under conditions of 37°C, 23°C, 4°C, and -80°C (in the dark) were significantly reduced compared to the amount of human AQP2 in the control sample, whereas the amount of human AQP2 in the urine preserved at -30°C was the same as the amount of human AQP2 in the control sample and no reduction in the quantity was observed.

### (2) The second test and its results

Each of the urine samples, which had been preserved under conditions of 374°C, 23° C, 4° C, -30° C, and -80° C (in the dark) and then returned to room temperature in the first test, was divided into three tubes; and these tubes were each preserved under conditions of 4°C, -30°C, and -80°C (in the dark) for 24 hours. After the preservation, each of the samples was left still under a room temperature condition to return to room temperature.

Using each of the samples as the test sample, the amount of human AQP2 in the urine was measured in a similar manner to that described above. The amount of human AQP2 obtained from the test sample preserved at -30°C in the first test was defined as 100, and a relative ratio of the amount of human AQP2 obtained for each preserved sample was determined.

As a result, as shown in FIG. 15, with respect to the test sample preserved at -30°C in the first test, the amount of human AQP2 in the urine was constant with substantially no change, whether the sample was preserved, in the second test, at -30°C or at a temperature condition (4°C or -80°C) other than -30°C. Moreover, even in the case of the test sample that was preserved at a temperature condition (37°C, 23°C , 4°C, or -804°C) other than -30°C in the first test, if the test sample was preserved at -30°C in the second test, the amount of human AQP2 in the urine became substantially 100. Thus, it was found that it is important for the AQP2 measurement to have the test sample preserved once at -30°C.

### (3) The third test, the fourth test, and the fifth test and their results

Each urine sample that was preserved under a condition of 4°C (in the dark) and returned to room temperature in the second test was divided into three tubes, and these tubes were preserved again under a condition of 4°C (in the dark) for 24 hours. After the preservation, each sample was left still under a room temperature condition to return to room temperature. Then, a portion of each sample was taken out (third test). The fourth test and the fifth test were performed in a similar manner.

Using each urine sample after these tests (the third test, the fourth test, and the fifth test) as the test sample, the amount of human AQP2 in the urine was measured in a similar manner to that described above. The amount of human AQP2 obtained from the test sample preserved at -30°C in the first test was defined as 100, and a relative ratio of the amount of human AQP2 obtained for each test sample above was determined.

As a result, as shown in FIG. 15, in the case where the preservation condition was 37° C, 23° C, 4° C, or -80° C, the relative ratio of the AQP2 value was 5 or lower, and thus AQP2 could not be detected. However, only if the -30°C condition was applied at least once, AQP2 was successfully detected. In the case of the test sample preserved at -30°C in the first test, no matter whether it was preserved at -30°C or at a temperature condition (4°C or -80°C) other than -30°C during the period from the second test to the fifth test, the amount of human AQP2 in the urine was hardly decreased and was stable. Moreover, it was confirmed that, even in the case of the test sample preserved under a temperature condition (37° C, 23° C, 4° C, or -80° C) other than -30°C in the first test or second test, if it is preserved at -30°C after the second test or the third test, the decrease in the amount of human AQP2 in the urine was significantly suppressed.

These result show that, in the case where a test sample, urine in particular, is subjected to an immunoassay (ELISA) of human AQP2 protein, it is possible to measure human AQP2 accurately at a high yield, by freezing, at least once, the test sample under a temperature condition of higher than -80°C, specifically -70°C to 0°C, in particular -30°C or a temperature near -30° C (-35°C to -15° C) .

### Experimental Example 3 Examination (No. 2) of pretreatment conditions for the test sample

In order to examine the pretreatment conditions for the test sample to be subjected to an immunoassay (ELISA) of human AQP2 protein established in Experimental Example 1, the following experiments were performed using urine as a test sample. As the urine, fresh urine obtained from a healthy human volunteer was used.

0.5 mL of the fresh urine was dispensed into each of seven Eppendorf tubes and frozen at a temperature of -20±5°C (in the dark) for 1 day, 3 days, 7 days, 15 days, 21 days, 28 days, and 56 days. After preservation, each sample was left still at room temperature to allow the sample to return to room temperature. Using each sample as a test sample, the AQP2 ELISA established in Experimental Example 1 was performed to measure the amount of human AQP2 in the urine.

FIG. 16 shows the results. As shown in FIG. 16, the amount of human AQP2 in all of the urine samples frozen at a temperature of -20±5°C for 3 days, 7 days, 15 days, 21 days, 28 days, and 56 days was successfully measured by ELISA. It was confirmed that when the urine (biological sample) is maintained in a frozen state for a certain period of time or longer, such as for about 2 weeks, the measured values are stabilized. These results indicate that in the immunoassay (ELISA) of human AQP2 protein in a test sample, in particular in urine, the human AQP2 protein in the biological sample can be quantitatively measured with high accuracy by maintaining the biological sample in a frozen state for about 2 weeks or longer.

Based on the above results, the freezing period was set to 2 weeks, and the effect of freezing temperatures (-80°C, -30°C, -28°C, -26°C, -20°C, 4°C) was investigated.

More specifically, using fresh urine samples (Nos. 1 and 2) obtained from two healthy human volunteers, 0.5 mL of each sample was dispensed into each of six Eppendorf tubes and preserved at -80°C, -30°C, -28°C, -26°C, -20°C, and 4°C (in the dark) for 2 weeks. After the preservation, each sample was left still at room temperature to allow the sample to return to room temperature. Using each sample as a test sample, the AQP2 ELISA established in Experimental Example 1 was performed to measure the amount of human AQP2 in the urine.

FIG. 17 shows the results. As shown in FIG. 17, the amounts of AQP2 in urine samples preserved at -80°C and 4°C (in the dark) were below the detection limit, whereas the amounts of AQP2 in the urine samples preserved at -30°C, -28°C, -26°C, and -20°C (in the dark) for 2 weeks were all successfully measured by ELISA.

The above results confirmed that when urine used in the immunoassay (ELISA) of human AQP2 protein is frozen under a freezing temperature condition of about -30°C to about -20°C for a period of about 2 weeks or longer, the amount of human AQP2 protein in the urine can be accurately measured in high yield.

### [Sequence List Free Text]

SEQ ID NOS. 2 and 3 are the base sequences of the forward primer (hAQP2N-F) and the reverse primer (hAQP2N-R) used to amplify a human AQP2 gene N-terminal portion (hAQP2 / first half portion, hAQP2N) by PCR. SEQ ID NOS. 4 and 5 are the base sequences of the forward primer (hAQP2C-F) and the reverse primer (hAQP2C-R) used to amplify a human AQP2 gene C-terminal portion (hAQP2 / second half portion, hAQP2C) by PCR.

## Claims

1. A method for pretreatment of a biological sample in the immunoassay of a protein contained in the biological sample, the method comprising the steps of:
(1) freezing the biological sample at a temperature higher than -80°C;
(2) thawing the frozen biological sample; and
(3) solubilizing the biological sample using a surfactant.

2. A method for pretreatment of a biological sample in the immunoassay of a protein contained in the biological sample, the method comprising the steps of:
(2') thawing a biological sample frozen at a temperature higher than -80°C; and
(3) solubilizing the biological sample using a surfactant.

3. The method for pretreatment according to claim 1 or 2, wherein the biological sample is a body fluid of an animal including a human.

4. The method for pretreatment according to claim 3, wherein the body fluid is urine.

5. The method for pretreatment according to any one of claims 1 to 4, wherein the surfactant used in solubilization step (3) is a nonionic surfactant.

6. The method for pretreatment according to claim 5, wherein the nonionic surfactant is polyoxyethylene(10) octylphenyl ether.

7. The method for pretreatment according to any one of claims 1 to 6, wherein the immunoassay is ELISA.

8. The method for pretreatment according to any one of claims 1 to 7, wherein the protein is a membrane protein.

9. An immunoassay for determining a protein contained in a biological sample, wherein the biological sample is pretreated by the method of any one of claims 1 to 8.

10. The immunoassay according to claim 9, which is an ELISA.

11. The immunoassay according to claim 9 or 10, wherein the biological sample is urine.

12. The immunoassay according to any one of claims 9 to 11, wherein the protein is a membrane protein.

13. The immunoassay according to claim 12, wherein the protein is aquaporin.
